# EUROPÄISCHE PATENTSCHRIFT

(11) **EP 0 970 249 B1**
(45) Veröffentlichungstag und Bekanntmachung des Hinweises auf die Patenterteilung: **27.11.2002**
(21) Anmeldenummer: 98919054.1
(22) Anmeldetag: 04.03.1998
(51) Int. Cl.: C12Q 1/68

(54) **VERFAHREN ZUM NACHWEIS VON MUTIERTEN ALLELEN**
METHOD FOR DETECTING MUTATED ALLELS
METHODE POUR DETECTER DES ALLELES MUTES

(30) Priorität: 04.03.1997 DE 19708758
(43) Veröffentlichungstag der Anmeldung: 12.01.2000
(73) Patentinhaber: Wagener, Christoph, 20251 Hamburg (DE)
(72) Erfinder: Wagener, Christoph, 20251 Hamburg (DE)
(74) Vertreter: Schüssler, Andrea, Dr.
(86) Internationale Anmeldenummer: DE9800676
(87) Internationale Veröffentlichungsnummer: WO98039472

(56) Entgegenhaltungen:
- EP-A- 0 461 496
- WO-A-90/09393
- WO-A-93/22457
- WO-A-95/12689
- US-A- 5 512 441
- DATABASE WPI Week 9211 Derwent Publications Ltd., London, GB; AN 92-085972 XP002079639 "Mutant detect nucleic acid compare lower hybrid stabilised sample standard nucleic acid base sequence" & JP 04 030800 A (TOSOH CORP) , 3. Februar 1992

## Beschreibung

Die vorliegende Erfindung betrifft ein Verfahren zum Nachweis von genetischen Veränderungen, insbesondere ein Verfahren zum Nachweis von wenigen mutierten Allelen in einem Überschuß von Wildtyp-Allelen.

Dem Nachweis punktmutierter Allele in einem Überschuß von Wildtyp-Allelen kommt ein bedeutendes diagnostisches Potential zu. Als Anwendungsbereiche sind beispielsweise zu nennen:
- Nachweis von Tumorzellen im Stuhl von Patienten mit Verdacht auf kolorektale Karzinome
- Nachweis von Tumorzellen in Sputum und Bronchiallavage von Patienten mit Verdacht auf Bronchialkarzinom
- Nachweis von Tumorzellen im Urin von Patienten mit Verdacht auf Blasenkarzinom
- Nachweis von Tumorzellen in entnommenen Gewebebiopsieproben

Bisher war nur die gezielte Amplifikation definierter Punktmutationen möglich, wobei es dafür notwendig war, die Punktmutätion bezüglich Ort und Aussehen genau zu kennen. Als Methoden können hierbei die allelspezifische Oligonukleotid-Hybridisierung klonierter PCR-Produkte (vgl. Sidransky, D. et al., Science 256, S. 102-105 (1992)) oder die "Mutant Enriched PCR" (vgl. Nollau, P. et al., Int. J. Cancer 66, S. 332-336 (1996)) angewendet werden. Diese Verfahren eignen sich aber nicht dazu, wenige punktmutierte Allele in einem Überschuß von Wildtyp-Allelen zu detektieren, wenn die Position der Mutation, z.B. Punktmutation, Deletion, im vorhinein nicht bekannt ist.

In den in WO-A-93/22457 und WO-A-95/12689 beschriebenen Verfahren werden Fehlpaarungen zwischen zwei DNA-Strängen durch sog. Mismatch-Bindungsproteine detektiert. Hierbei handelt es sich um Proteine, die Bestandteile des sog. Mismatch-Reparatur-Systems sind.

Die Aufgabe der vorliegenden Erfindung bestand deshalb darin, ein Verfahren bereitzustellen, mit dem wenige mutierte Allele in einem Überschuß von Wildtyp-Allelen nachgewiesen und abgetrennt werden können.

Die Aufgabe wird durch ein Verfahren gemäß Patentanspruch 1 gelöst. Vorteilhaffe Ausgestaltungen ergeben sich aus den Unteransprüchen.

Zu einem "mutierten Allel" kommt es, wenn im Vergleich zum Wildtyp Punktmutationen, Deletionen, Insertionen, Inversionen bzw. Substitutionen kleinerer oder größerer Genbereiche auftreten.

Untersuchungsproben, die sich für die Analyse auf das Verhandensein von mutierten Genen eignen, sind z.B.: Blut, Urin, Stuhl, Speichel, Sputum, Bronchiallavage, Abstrichmaterial und Biopsiematerial.

Die Erfindung beruht auf dem Prinzip einer allelspezifischen Oligonukleotid-Hybridisierung. Dafür werden Oligonukleotide von 12-25, bevorzugt 16-20, Basenpaare (bp) an ein geeignetes Trägermaterial gebunden, wobei die Oligonukleotide als Sonden zu Abschnitten des Wildtyp-Allels komplementär sind. Die Oligonukleotid-Sonden liegen im Vergleich zu den Zielsequenzen in hohem Überschuß vor.

Geeignete Trägermaterialien, an die die Oligonukleptide gebunden werden, sind beispielsweise Glas, wie Silikate, Gelmaterialien, wie Agarose und Dextran, oder Polymermaterialien, wie Polypropylen oder Polyacrylamid. Die Bindung der Oligonukleotide erfolgt vorzugsweise kovalent, da eine adsorptive Bindung in den seltensten Fällen fest genug ist. Verfahren zur kovalenten Bindung von Oligonukleotiden an Trägeroberflächen sind beispielsweise in Khrapko, K.R. et al., DNA Seq. 1, S. 375-388 (1991); Fodor S.P.A. et al., Science 251, S. 767-773 (1991) und Maskos et al., Nucl. Acid Research 20, S. 1639-1648 (1992) beschrieben.

Die Untersuchungsprobe, die sowohl Wildtyp- (im Überschuß) als auch mutierte Allele (wenig) enthält, wird nun einem Trennverfahren, z.B. Chromatographie oder Elektrophorese, über den Träger, an den die Oligonukleotide bevorzugt kovalent gebunden sind, unterworfen. Die Bedingungen zur Durchführung der Trennverfahren sind dem Fachmann geläufig und es werden gebräuchliche Trennvorrichtungen, beispielsweise in Form von Chromatographiesäulen, Elektrophoresekammern, -röhrchen oder -kapillaren eingesetzt. Für die Hybridisierung des Wildtyp-Allels mit dem gebundenen Oligonukleotid muß die ggf. amplifizierte DNA einzelstängig vorliegen. Dies kann z.B. durch Puffer mit entsprechendem Salzgehalt, z.B. SSC oder SSPE, erreicht werden. Besser geeignet ist die vorherige Abtrennung eines DNA-Strangs über einen entsprechend markierten Primer. Bei Verwendung z.B. eines Biotin-markierten Primers kann die Abtrennung des betreffenden DNA-Strangs über die Bindung an Streptavidin-Beads erfolgen.

Da die Oligonukleotide nur zu den Sequenzen des Wildtyps komplementär sind, nicht zu denen der Mutante, hybridisieren sie entweder ausschließlich mit dem Wildtyp-Allel oder retardieren dessen Mobilität selektiv. Bei ausschließlicher Bindung des Wildtyps finden sich die mutierten Fragmente in der nicht an die Oligonukleotide auf dem Träger gebundenen Probenfraktion, bei bevorzugter Bindung des Wildtyps wird das mutierte Fragment vor dem Wildtyp-Fragment eluiert und damit davon abgetrennt.

Die Sensitivität des Verfahrens kann weiter erhöht werden, wenn zusätzlich zum Sense-Strang des Wildtyp-Allels der anti-Sense-Strang in die Analytik einbezogen wird.

Methodisch am einfachsten ist der Fall, wenn ein Allel nur Punktmutationen aufweist und die Stellen, an denen diese gewöhnlich auftreten, bekannt sind. Dies ist beispielsweise beim K-ras-Gen der Fall, das bei kolorektalen Karzinomen ausschließlich Mutationen im Kodon 12 oder 13 aufweist. In diesem Fall muß nur ein Oligonukleotid von ca. 20 bp synthetisiert werden, das komplementär zu dem Bereich des Wildtyp-Allels ist, in dem Kodon 12 und 13 liegen. Das Oligonukleotid wird an einen Träger gebunden, der für Trennzwecke, z.B. chromatographische oder elektrophoretische Zwecke, geeignet ist. Das interessierende Gen, hier: K-ras-Gen, wird aus der Untersuchungsprobe isoliert und in Form von Restriktionsfragmenten oder PCR-Produkten, die die Kodons 12 und 13 umspannen, markiert, z.B. mittels Radionukliden, Fluoreszenzfarbstoffen, Biotin/-Avidin-System, und dem gewählten Trennverfahren unterworfen. Für das Trennverfahren ist es vorteilhaft, wenn die DNA einzelsträngig vorliegt. Dies kann z.B. dadurch erreicht werden, daß einer der beiden DNA-Stränge mit Biotin markiert wird und über die Bindung an Avidin abgetrennt wird. Bei ausschließlicher Bindung des Wildtyps finden sich die mutierten Fragmente in der nicht gebundenen Fraktion und können nach Auffangen analysiert werden. Bei bevorzugter Bindung des Wildtyps wird ein Puffer angewendet, der das mutierte Fragment vor dem Wildtyp-Fragment vom Träger eluiert. Hier müssen Salzlösungen und Temperatur so gewählt sein, daß das Wildtyp-Allel im Vergleich zum mutierten Allel retardiert wird. Als Salz ist Tetramethylammoniumchlorid besonders geeignet, da die Stabilität von CG- und AT-Basenpaarungen vergleichbar ist. Die Temperatur sollte im Bereich der Schmelztemperatur (Tm) des Wildtyp-Allels liegen. Bei Verwendung von z.B. 20mer gebundenen Oligonukleotiden und 3,0M Tetramethylammoniumchlorid liegt die Schmelztemperatur eines vollständig komplementären Hybrids bei 60°C.

Methodisch anders vorzugehen ist in dem Fall, wo es um den Nachweis für Gene mit multiplen, heterogenen Punktmutationen geht. Als Beispiel kann das p53-Gen genannt werden. Im p53-Gen finden sich multiple Mutationen, die über verschiedene Exons verteilt sind. Um das Problem des Mutationsnachweises im p53-Gen und bzgl. des Mutationstyps vergleichbarer Gene zu lösen, sind prinzipiell folgende Wege möglich:
- Parallelschaltung von Trennvorrichtungen, z.B. Säulen oder Kapillaren, an deren Trennmaterial (Träger) jeweils verschiedene Oligonukleotide gebunden sind
- Serienschaltung von Trennvorrichtungen, an deren Trennmaterial jeweils verschiedene Oligonukleotide gebunden sind
- Bindung von Oligonukleotiden, die mit verschiedenen Abschnitten eines Gens hybridisieren, an einen Träger

### Parallelschaltung von Trennvorrichtungen

Es werden Trägermateriallen für Trennvorrichtungen eingesetzt, an die ein Oligonukleotid gebunden ist, das zu einem bestimmten Bereich des Wildtyp-Allels komplementär ist. Wenn Sense- und anti-Sense-Strang in die Analytik einbezogen werden, sind für einen bestimmten Genabschnitt jeweils zwei Trennvorrichtungen mit den entsprechenden komplementären Oligonukleotid erforderlich. Jede Trennvorrichtung enthält ein Oligonukleotid (bzw. zwei Oligonukleotide bei Verwendung von sense und anti-sense), dessen Basensequenz von derjenigen der Oligonukleotide anderer Trennvorrichtungen verschieden ist. Bei einer Oligonukleotidlänge von 20 bp müßten z.B. mindestens 30 Trennvorrichtungen hergestellt werden, um den gesamten Bereich einer Ziel-Wildtypsequenz von 600 bp abzudecken. Nach PCR-Amplifikation oder entsprechendem Restriktionsverdau unter Erhalt einer solchen Zielsequenz mit 600 bp wird diese gemäß Standardmethoden in Einzelstrang umgewandelt, markiert, z.B. mit Fluoreszenzfarbstoffen, Radionukliden oder Avidin/Biotin-System, und die 30 Trennvorrichtungen parallel beladen. Das mutierte Allel wird von dem Wildtyp-Allel durch die Trennvorrichtung abgetrennt, die mit einem Träger arbeitet, der einen Mismatch zum mutierten Allel aufweist. Durch Portionierung der zu untersuchenden Probe wird bei Parallelschaltung der Trennvorrichtungen verglichen mit der Serienschaltung die Sensitivität etwas vermindert.

### Serienschaltung von Trennvorrichtungen

In diesem Fall werden die Trennvorrichtungen, die jeweils ein bestimmtes, von den Oligonukleotiden der anderen Kammern verschiedenes Oligonukleotid enthalten, hintereinander geschaltet. Bei diesem Vorgehen wird vorausgesetzt, daß die Wildtyp-Fragmente quantitativ binden und die mutierten Fragmente überhaupt nicht binden. Zwischen jeder Trennvorrichtung ist jeweils ein Ventil angebracht. Je nach Stellung des Ventils wird eine Meßzelle (Ventilstellung I) oder die nächste Trennvorrichtung (Ventilstellung II) beladen. Als Meßzelle kommt z.B. ein Fluoreszenzphotometer oder ein Szintillationsmeßgerät in Frage. Die Elution gebundener Fragmente erfolgt gemäß Standardmethoden, wie mittels Hitze oder Änderung der Salzkonzentration. Der Trennvorgang vollzieht sich beispielsweise wie folgt:
Ventil nach Trennvorrichtung 1: Stellung I
Beladung von Trennvorrichtung 1
Beladung der Meßzelle durch nicht-gebundene Fraktion
Ventil nach Trennvorrichtung 1: Stellung II
Ventil nach Trennvorrichtung 2: Stellung I
Beladung von Trennvorrichtung 2
Beladung der Meßzelle durch nicht-gebundene Fraktion
usw.

Die Serienschaltung bietet im Vergleich zur Parallelschaltung den potentiellen Vorteil einer höheren Sensitivität.

### Bindung von Oligonukleotiden, die mit verschiedenen Abschnitten des Wildtyp-Allels hybridisieren, an einen Träger

Prinzipiell funktioniert auch eine Trennung, wenn Oligonukleotide, die zu verschiedenen Abschnitten der Ziel-Wildtypsequenz komplementär sind, an einen Träger gekoppelt werden. Hier müssen die Pufferbedingungen so gewählt werden, daß das Wildtyp-Allel im Vergleich zum mutierten Allel retardiert wird. Dies kann dann erreicht werden, wenn die Trennung bei einer Temperatur erfolgt, die im Bereich der Schmelztemperatur liegt bzw. um die Schmelztemperatur oszilliert. Unter diesen Bedingungen kommt es zu einer reversiblen Interaktion zwischen gebundenen Oligonukleotiden und Wildtypsequenzen. Derjenige Bereich des Gens, der eine Punktmutation aufweist, wird nicht gebunden. Insgesamt ist somit die Interaktion zwischen mutierten Allelen und gebundenen Oligonukleotiden schwächer als die Interaktion zwischen Wildtyp-Allel und gebundenen Oligonukleotiden. Das Prinzip der Trennung impliziert, daß die Trennleistung mit zunehmender Zahl unterschiedlicher gebundener Oligonukleotide abnimmt.

Die vorliegende Erfindung zeichnet sich dadurch aus, daß wenige mutierte Allele in einem Überschuß von Wildtyp-Allelen nachgewiesen werden können. Ferner eignet sie sich, heterozygote und homozygote Mutationen bzw. Polymorphismen nachzuweisen. Die vorliegende Erfindung stellt somit ein Mittel bereit, genetische Veränderungen vielfältigen Ursprungs zu analysieren. Daher findet die vorliegende Erfindung breite Anwendung in der Diagnostik. Desweiteren liefert sie die Basis für die Entwicklung neuer therapeutischer Ansätze.

Die Erfindung wird weiter anhand der Figuren verdeutlicht, die zeigen:
- **Fig. 1**:: Trennung mutierter *p53*-Allele von *p53*-Wildtypallelen durch parallel geschaltete Trennkammern mit immobilisierten Oligonukleotiden
- **Fig. 2a**:: Ventilstellungen bei der seriellen Schaltung von Trennkammern
- **Fig. 2b**:: Trennung mutierter *p53*-Allele von *p53*-Wildtypallelen durch seriell geschaltete Trennkammern mit immobilisierten Oligonukleotiden.

Die Erfindung wird nun weiter anhand der Beispiele erläutert.

### BEISPIEL 1: Analyse einer Mutation im K-ras-Gen

DNA kann aus Geweben, Körperflüssigkeiten, Sekreten oder Exkreten extrahiert werden. Hierzu werden Proben, z.B. Stuhl, Blut, Pankreassaft, Urin oder Sputum, in einer wäßrigen Lösung von 6 M Guanidinum Isothiocyanat suspendiert. Nach Zentrifugation wird NP-40 hinzugegeben (Endkonzentration 1 %). Nach einer Inkubationszeit von mindestens 10 min. bei Raumtemperatur werden 500 µl der Suspension in eine kommerziell erhältliche Patrone mit einem Glasfilter zur Isolierung von DNA überführt. Nach Zentrifugation und zweimaligem Waschen mit kaltem Ethanol (4°C) wird die DNA mit heißem Wasser (70°C) eluiert. Um eine Degradation zu verhindern, erfolgt eine längere Lagerung in 10 mM Tris-HCI (pH 7,4).

Zur Durchführung einer PCR-Amplifikation werden 500 ng DNA in 100 µl eines 10 mM Tris-HCI-Puffers, pH 8,3 überführt. Der Puffer enthält folgende Zusätze: 1,5 mM MgCl₂, 50 mM KCI 0,01 % (w/v) Gelatine, jweils 200 µM dNTP, 2,5 U *Taq* Polymerase und 0,3 µMol der jeweiligen Primer. Die Sequenz der Primer ist wie folgt: Sense: 5'-GTATTAACCTTATGTGTGACATGTTC-3'; anti-Sense:
5'-TCAAAGAATGGTCCTGCACC-3'. In den anti-Sense-Primer wird zum Abschluß der Oligonukleotid-Synthese in einem automatischen DNA-Synthesizer am 5'-Ende ein biotinyliertes Nukleotid, in den Sense-Primer am 5'-Ende ein mit einem Fluoreszenz-Farbstoff (z.B. Fluorescein) markiertes Nukleotid eingebaut. Die markierten Nukleotide sind kommerziell erhältlich.

Zum Nachweis von Mutationen in Kodons 12 und 13 des K-*RAS*-Gens wird ein 20mer Oligonukleotid an feste Träger synthetisiert bzw. an feste Träger gekoppelt. Die Sequenz des Oligonukleotids ist wie folgt:
5'-GCCTACGCCACCAGCTCCAA-3'. Als feste Träger kommen alle für chromatographische und elektrophoretische Trennung geeigneten Träger in Frage. Glas und Polyacrylamid seien beispielhaft als Trennmedien aufgeführt.

Als Glasträger sind z.B. poröse Kugeln geeignet. Die Derivatisierung der Glasoberfläche erfolgt nach üblichen Verfahren, die Synthese der Oligonukleotide erfolgt direkt auf den derivatisierten Glasoberflächen in einem automatischen DNA Synthesizer (Applied Biosystems) nach Vorschrift des Herstellers. Zur Derivatisierung werden die Glaskugeln (10 g) in 40 ml Xylol + 12 ml 3-Glycidoxypropyltrimethoxysilan mit einer Spur Hünig-Base bei 80°C für ca. 12 Std. inkubiert. Nach Waschen in Methanol und Ether werden die Kugeln in Luft und unter Vakuum getrocknet. In einem zweiten Schritt wurden Alkyl-Spacermoleküle an die derivatisierte Oberfläche gekoppelt. Hierzu werden die Kugeln z.B. in Pentaethylenglycol überführt. Nach Waschen in Methanol und Ether werden die Kugeln in Luft und unter Vakuum getrocknet. Die Lagerung erfolgt unter Argon bei -20°C (Maskos U. & Southern EM. Nucleic Acids Res. 10, 1679-1684 (1992)).

Die so derivatisierten Glaskugeln werden direkt in den DNA-Synthesizer eingesetzt.

Zur Kopplung an Polyacrylamid wird bei der Oligonukleotidsynthese am 3'-Ende eine Methyluridin-Base eingebaut. In das Polyacrylamidgel werden durch Behandlung mit einer 50 %igen wäßrigen Hydrazinhydrat-Lösung (1 Std., Raumtemperatur) Hydrazingruppen eingeführt. Zur Kopplung wird die Ribose am 3'-Ende des Oligonukleotids mit Natriumperiodat oxidiert. Die entstehende Aldehydgruppe wird an das derivatisierte Gel gebunden (Khrapko K.R. et al., DNS Seq. 1, 375-388 (1991)).

Um eine Rehybridisierung der DNA-Einzelstränge im Verlauf von Chromatographie oder Elektrophorese zu verhindern, ist es sinnvoll, einzelsträngige DNA für die Trennung vorzusehen. Hierzu ist der Primer im anti-Sense-Strang biotinyliert. Das PCR-Produkt wird erhitzt und über eine Streptavidin-Festphase passiert (z. B. Dynabeads). Auf diese Weise wird der anti-Sense-Strang entfernt. Soll der anti-Sense-Strang analysiert werden, kann auch der Sense-Strang über einen entsprechenden biotinylierten Primer entfernt werden.

Die Trennung von Wildtyp und in Kodon 12 bzw. 13 des K-ras Gens mutierten Amplifikaten erfolgt z.B. über Säulenchromatographie oder Kapillarelektrophorese (Acrylamidgel-Füllung der Kapillaren). Bei Beladung des Trägers werden Pufferund Temperaturbedingungen so gewählt, daß die Schmelztemperatur über derjenigen mutanter Allele und unter derjenigen von Wildtyp-Allelen liegt. Als Puffer kommen z.B. SSC oder SSPE in entsprechender Konzentration (5-4x) bzw. 3 M Tetramethylammoniumchlorid-Lösung in Frage. Über einen Heizblock muß für Temperaturkonstanz in der Trennvorrichtung gesorgt werden (z.B. 58°C bei Verwendung von 20mer Oligonukleotiden und 3 M Tetramethylammoniumchlorid-Lösung).

Die Detektion der Fluoreszenz-markierten Amplifikate geschieht mittels Laserinduzierter Fluoreszenz unter Verwendung z.B. eines Argon-Lif-Detektors. Zur empfindlichen Detektion der DNA kann auch eine zweite PCR angeschlossen werden.

### BEISPIEL 2: Nachweis von Punktmutationen im 5. Exon des p53-Gens

Die DNA wird, wie in Beispiel 1 beschrieben, isoliert. Zur Amplifikation des 5. Exons werden folgende Primer verwendet:
Sense: 5'-TTTCAACTCTGTCTCCTTCC-3';
anti-Sense:AACCAGCCCTGTCGTCTCTC-3'. Da die Primer in Introns liegen, kann die gesamte Sequenz von Exon 5 analysiert werden. Der anti-Sense-Primer wird am 5'-Ende mit einem biotinylierten Oligonukleotid markiert, der Sense-Primer am 5'-Ende mit einem Fluoreszenz-markierten Oligonukleotid. Die PCR erfolgt wie vorstehend beschrieben. Der biotinylierte DNA-Strang wird durch Bindung an immobilisiertes Streptavidin abgetrennt.

### (a) Trennung mittels parallel-geschalteter Trennvorrichtungen

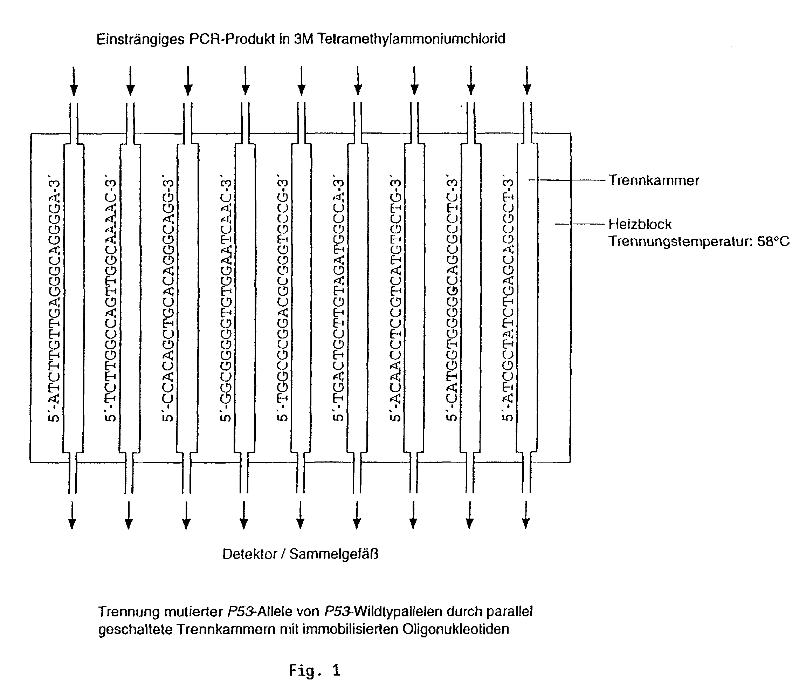

Die Trennvorrichtung besteht aus Säulen mit einem definierten Trennmedium, z.B. Glaskugeln, Polyacrylamid (Abb.1). An das Trennmedium in einer bestimmten Säule wird ein definiertes 20mer Oligonukleotid, wie vorstehend beschrieben, kovalent gebunden. Das Oligonukleotid ist zu einem gegebenen Abschnitt der DNA von Exon 5 des *p53*-Gens komplementär. Die in den verschiedenen Säulen an das Trennmedium gebundenen Oligonukleotide decken insgesamt die gesamte Sequenz des 5. Exons ab. (s. Abb. 1). Die Säulen befinden sich in einem Heizblock, um konstante Temperatur zu gewährleisten. Die Temperatur für die Trennung von Wildtyp- und mutiertem Allel beträgt 58°C. Die Säulen sind mit 3 M Tetramethylammoniumchlorid-Lösung äquilibriert. Das zu untersuchende PCR-Produkt, das Wildtyp- und mutiertes Allel enthält, ist ebenfalls in dieser Lösung gelöst. Ferner ist das PCR-Produkt einzelsträngig (Sense-Strang) und Fluoreszenz-markiert. Es wird in Aliquots auf die Säulen aufgetragen. Das mutierte Alle weist einen Mismatch zum Oligonukleotid in der dritten Trennkammer von links auf. Das mutierte Allel liegt im Vergleich zu den Wildtyp-Allelen im Unterschuß vor. Die Wildtyp-Allele werden in sämtlichen Säulen gebunden. Das mutierte Allel passiert die dritte Säule von links. Es wird über das Fluoreszenzsignal detektiert.

Säulen oder Detektor können beweglich sein. Vor der Beladung einer definierten Säule wird der Detektor mit dem Auslaß der Säule verbunden. Nach Ablauf der Reaktion wird der Detektor mit der nächsten Säule verbunden. Hierdurch kann das Fluoreszenzsignal einer bestimmten Säule und die Mutation somit einen definierten Abschnitt der DNA-Sequenz zugeordnet werden. Dies ermöglicht den gezielten Nachweis einer Mutation nach einer zweiten PCR, z.B. durch allelspezifische Oligonukleotid-Hybridisierung oder DNA-Sequenzierung.

### (b) Trennung mittels seriell geschalteter Trennkammern

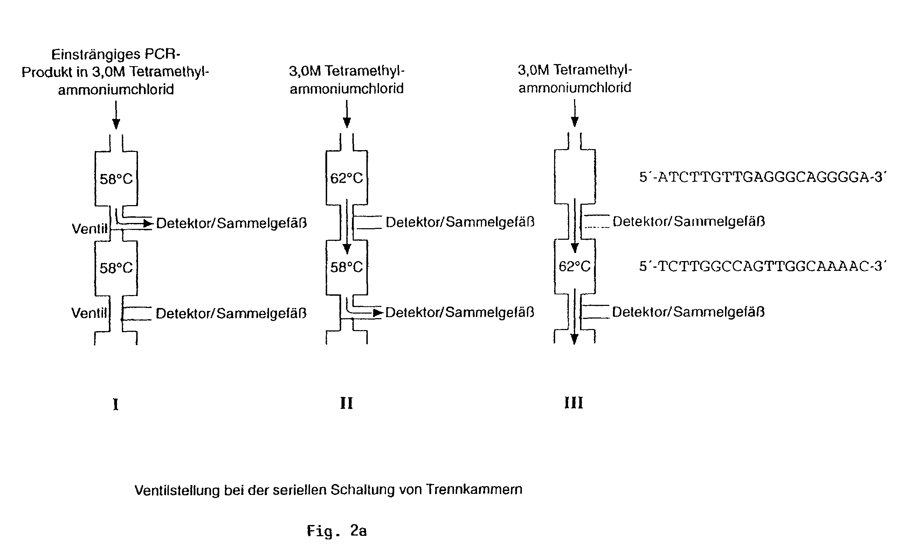

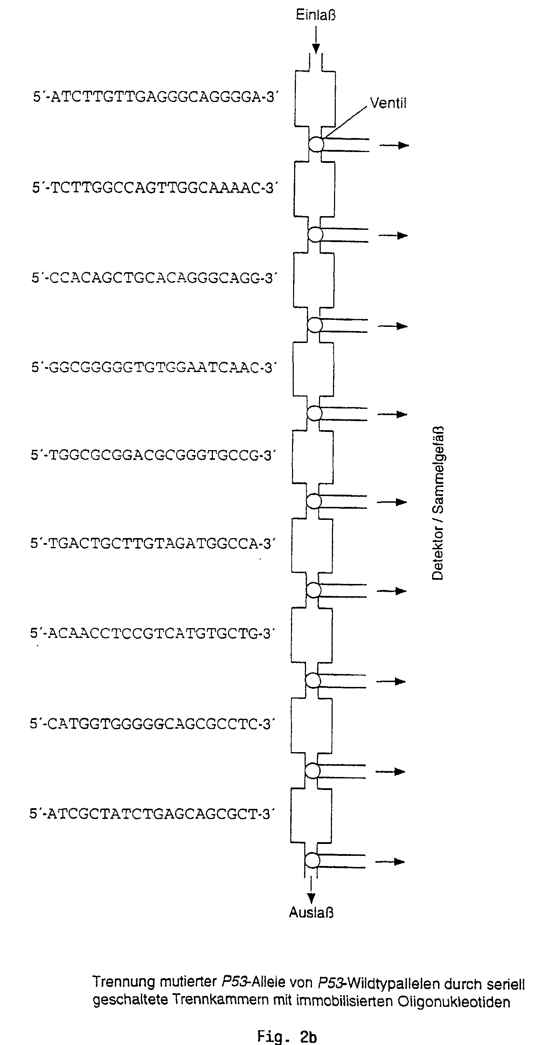

Es wird eine säulenchromatographische Trennung von in Serie geschalteten Trennkammern beschrieben. Die einzelnen Säulen sind, wie unter (a) beschrieben, mit einem Trennmedium gefüllt, an welches pro Säule ein definiertes Oligonukleotid gebunden ist. Die in den verschiedenen Säulen gebundenen Oligonukleotide decken wiederum die gesamte Sequenz von Exon 5 ab. Die Säulen sind temperierbar. Bei serieller Schaltung befindet sich zwischen den einzelnen Säulen ein Ventil. Beladung und Ventilstellung sind in Abb. 2a schematisch wiedergegeben. Die an die Trennmatrix gebundenen Oligonukleotide sind rechts angegeben. Fluoreszenzmarkierung, Isolierung der einzelsträngigen DNA und Säulen bzw. Probenlösung sind wie unter (a) beschrieben. Vor der Probenaufgabe verschließt das Ventil zwischen Säule 1 und 2 den Zugang zu Säule 2 und öffnet den Zugang zu Detektor oder Sammelgefäß (Stellung I). Die Temperatur in Säule 1 beträgt 58°C. Das einzelsträngige PCR-Produkt passiert Säule 1. Wildtyp-DNA wird gebunden. Falls sich in dem zum gebundenen Oligonukleotid komplementären DNA-Abschnitt des mutierten Allels eine Punktmutation befindet, wird das mutierte Allel nicht gebunden und dem Detektor bzw. Sammelgefäß zugeführt. Falls sich die Punktmutation in einem anderen DNA-Abschnitt befindet, wird das mutierte Allel ebenfalls gebunden. Nach Abschluß der Reaktion wird die Ventilstellung zwischen Säule 1 und 2 so verändert, daß der Zugang zu Säule 2 freigegeben und der Ausgang zu Detektor/Sammelgefäß geschlossen wird. Das Ventil zwischen Säule 2 und 3 verschließt den Zugang zu Säule 3 und öffnet den Zugang zu Detektor/Sammelgefäß (Ventilstellungen II). Säule 2 ist auf 58°C temperiert. Säule 1 wird auf eine Temperatur gebracht, die über der Tm des Wildtyps liegt. Anschließend wird Puffer durch das System gepumpt. Die gebundene DNA wird von Säule 1 eluiert. In Säule 2 hybridisiert die Wildtyp-DNA mit dem auf der rechten Seite dargestellten Oligonukleotid. Falls sich in dem zum gebundenen Oligonukleotid komplementären DNA-Abschnitt des mutierten Allels eine Punktmutatione befindet, wird das mutierte Allel nicht gebunden und dem Detektor bzw. Sammelgefäß zugeführt. Falls sich die Punktmutation in einem anderen DNA-Abschnitt befindet, wird das mutierte Allel ebenfalls gebunden. Anschließend wird die Ventilstellung zwischen Säule 2 und 3 so geändert, daß der Zugang zu Säule 3 geöffnet und der Zugang zu Detektor/Sammelgefäß geschlossen ist (III). Die Proben-DNA wird durch Erhitzen eluiert und in Säule 3 überführt. Der Vorgang wird entsprechend weitergeführt, bis alle Säulen passiert wurden. In Abb. 2b ist eine Trennvorrichtung dargestellt, die den Nachweis von Punktmutationen im gesamten Exon 5 des *p53-*Gens ermöglicht. Die auf der linken Seite dargestellten gebundenen Oligonukleotide decken den gesamten Bereich des Sense-Strangs von Exon 5 des *p53*-Gens ab.

## Patentansprüche

1. Verfahren zum Nachweis von mutierten Allelen in einem Überschuß von Wildtyp-Allelen, umfassend die Abtrennung der einzelsträngig gemachten Wildtyp-Allele in einem Trennverfahren über einen Träger, an den ein oder mehrere zu dem Wildtyp-Allel komplementäre Oligonukleotide gebunden sind, wobei das Trennprinzip darauf beruht, daß die Oligonukleotide entweder ausschließlich mit dem Wildtyp-Allel hybridisieren oder dessen Mobilität selektiv retardieren.

2. Verfahren nach Anspruch 1, wobei das Trennverfahren Chromatographie oder Elektrophorese ist.

3. Verfahren nach Anspruch 1 oder 2, wobei das Trennverfahren Kapillarelektrophorese ist.

4. Verfahren nach einem der Ansprüche 1 bis 3, wobei das mutierte Allel eine oder mehrere Punktmutationen, Deletionen, Inversionen, Insertionen und/oder Substitutionen kleiner oder größerer Genbereiche aufweist.

5. Verfahren nach einem der Ansprüche 1 bis 4, wobei für das Trennverfahren mehrere Trennvorrichtungen parallel oder in Serie geschaltet sind, wobei jede Trennvorrichtung ein Oligonukleotid enthält, dessen Basensequenz von derjenigen der Oligonukleotide anderer Trennvorrichtungen verschieden ist.

6. Verfahren nach einem der Ansprüche 1 bis 5, wobei zu dem Wildtyp-Allel komplementäre sense- und/oder anti-sense Oligonukleotide verwendet werden.

7. Verfahren nach einem der Ansprüche 1 bis 6, wobei die Oligonukleotide 16 bis 20 Basenpaare umfassen.

8. Verwendung des Verfahrens nach einem der Ansprüche 1 bis 7 in der Diagnostik von genetischen Veränderungen.

9. Verwendung nach Anspruch 8, wobei es sich um Krebsdiagnostik handelt.

## Claims

1. A method of detecting mutated alleles in an excess of wild-type alleles, comprising separating the wild-type alleles rendered single-stranded in a separating method via a carrier to which one or more oligonucleotides complementary to the wild-type allele are bound, the separating principle being based on the fact that the oligonucleotides either hybridize exclusively with the wild-type allele or retard selectively the mobility thereof.

2. The method according to claim 1, wherein the separating method is chromatography or electrophoresis.

3. The method according to claim 1 or 2, wherein the separating method is capillary electrophoresis.

4. The method according to any of claims 1 to 3, wherein the mutated allele has one or more point mutations, deletions, inversions, insertions and/or substitutions of small or relatively large gene regions.

5. The method according to any of claims 1 to 4, wherein for the separating method several separating devices are connected in parallel or in series, each separating device containing an oligonucleotide whose base sequence differs from that of the oligonucleotides of other separating devices.

6. The method according to any of claims 1 to 5, wherein sense and/or antisense oligonucleotides complementary to the wild-type allele are used.

7. The method according to any of claims 1 to 6, wherein the oligonucleotides comprise 16 to 20 base pairs.

8. Use of the method according to any of claims 1 to 7 for the diagnosis of genetic modifications.

9. Use according to claim 8, for the diagnosis of cancer.

## Revendications

1. Méthode de détection d'allèles mutés dans un excédent d'allèles de type sauvage, comprenant la séparation des allèles de type sauvage rendus monocaténaires dans une opération de séparation sur un support auquel sont liés un ou plusieurs oligonucléotides complémentaires de l'allèle de type sauvage, le principe de séparation reposant sur le fait que les oligonucléotides ou bien s'hybrident exclusivement avec l'allèle de type sauvage, ou bien retardent sélectivement sa mobilité.

2. Méthode suivant la revendication 1, dans laquelle l'opération de séparation est une chromatographie ou une électrophorèse.

3. Méthode suivant la revendication 1 ou 2, dans laquelle l'opération de séparation est une électrophorèse capillaire.

4. Méthode suivant l'une des revendications 1 à 3, dans laquelle l'allèle muté présente une ou plusieurs mutations ponctuelles, délétions, inversions, insertions et/ou substitutions de régions géniques plus petites ou plus grandes.

5. Méthode suivant l'une des revendications 1 à 4, dans laquelle plusieurs dispositifs de séparation sont montés en parallèle ou en série pour l'opération de séparation, chaque dispositif de séparation contenant un oligonucléotide dont la séquence de bases est différente de celle des oligonucléotides d'autres dispositifs de séparation.

6. Méthode suivant l'une des revendications 1 à 5, dans laquelle des oligonucléotides, sens et/ou antisens complémentaires de l'allèle de type sauvage sont utilisés.

7. Méthode suivant l'une des revendications 1 à 6, dans laquelle les oligonucléotides comprennent 16 à 20 paires de bases.

8. Utilisation de la méthode suivant l'une des revendications 1 à 7 dans le diagnostic de modifications génétiques.

9. Utilisation suivant la revendication 8, dans laquelle il s'agit de diagnostic du cancer.
